# EUROPÄISCHE PATENTSCHRIFT

(11) **EP 0 922 038 B1**
(45) Veröffentlichungstag und Bekanntmachung des Hinweises auf die Patenterteilung: **06.02.2002**
(21) Anmeldenummer: 96927636.9
(22) Anmeldetag: 31.07.1996
(51) Int. Cl.: C07D 251/68

(54) **VERFAHREN ZUR HERSTELLUNG VON SUBSTITUIERTEN 4,4'-DIAMINOSTILBEN-2,2'-DISULFONSÄURESALZEN**
PROCESS FOR PREPARING SUBSTITUTED 4,4'-DIAMINOSTILBENE-2,2'-DISULPHONIC ACID SALTS
PROCEDE DE PREPARATION DE SELS SUBSTITUES DE 4,4'-DIAMINOSTILBENE-2,2'-ACIDE DISULFONIQUE

(43) Veröffentlichungstag der Anmeldung: 16.06.1999
(73) Patentinhaber: BAYER AG, 51368 Leverkusen (DE)
(72) Erfinder: FELDHUES, Ulrich, D-51465 Bergisch Gladbach (DE); ECKSTEIN, Udo, D-51061 Köln (DE); VOGT, Uwe, D-40789 Monheim (DE); BROCKMANN, Rolf, D-51467 Bergisch Gladbach (DE)
(86) Internationale Anmeldenummer: EP9603357
(87) Internationale Veröffentlichungsnummer: WO9805653

(56) Entgegenhaltungen:
- GB-A- 1 243 479
- GB-A- 1 355 218
- US-A- 3 522 692
- US-A- 3 558 611

## Beschreibung

Die Erfindung betrifft ein verbessertes Verfahren zur Herstellung von Verbindungen der Formel 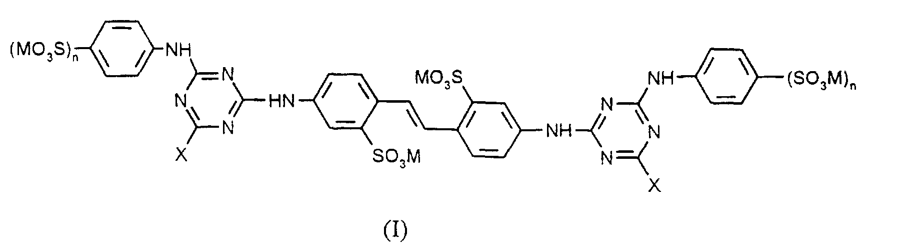 worin
- n: für 0 oder 1 steht,
- M: für ein Alkalimetallion oder ein gegebenenfalls substituiertes Ammoniumion und
- X: für N-Alkylamino oder N,N-Dialkylamino stehen, wobei im Fall von N,N-Dialkylamino die beiden gegebenenfalls durch ein Heteroatom aus der Reihe O, N, S unterbrochenen Alkylreste gemeinsam mit dem N-Atom, an das sie gebunden sind, einen gesättigten 5- oder 6-gliedrigen Heterocyclus bilden können.

Die Verbindungen (I) sind wichtige optische Aufheller, u.a. für Polyamid, Cellulose, Papier und Waschmittel.

Bevorzugte Alkalimetallionen sind Natrium- und Kaliumionen, bevorzugtes Ammoniumion ist das Triethanolammoniumion.

Unter Alkylresten in den zusammengesetzten Begriffen N-Alkylamino und N,N-Dialkylamino werden Alkylreste mit bis zu 4 C-Atomen verstanden, die durch ein O-Atom unterbrochen sein können und/oder Hydroxy, Cyano, Carbamoyl oder Sulfo als Substituenten tragen können.

Beispiele für gesättigte 5- und 6-gliedrige Heterocyclen, die aus den beiden Alkylresten der N,N-Dialkylaminogruppe und dem Stickstoffatom, an das sie gebunden sind, gebildet werden können, sind beispielsweise Pyrrolidin, Piperidin, N-Methylpiperazin, N-2-Hydroxyethylpiperazin und insbesondere Morpholin.

Ein ähnliches Verfahren wird in GB-A-1 355 218 beschrieben. Verfahren zur Herstellung von Verbindungen (I) aus Cyanurchlorid, einem 4,4'-Diaminostilben-2,2'-disulfonsäuresalz der Formel 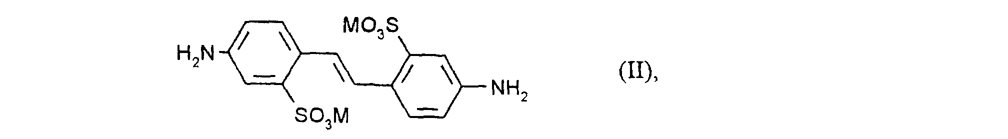 einem Anilin der Formel 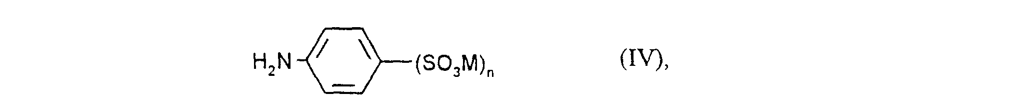 worin M und n die obengenannten Bedeutungen haben,
und einem Amin XH (VI), wobei X jeweils die in (I) angegebene Bedeutung besitzt, sind beschrieben; DE-AS 1 250 830, GB-PS 1 114 021 und 1 174 631, US-PS 3 532 692, PL-PS 76 705, RO-PS 62 947, DD-PS 154 778 und 159 334, CS-PS 228 871, JP-PS 62/106 965.

Überraschenderweise wurde nun gefunden, daß sich unerwartete Vorteile einstellen, wenn man die Umsetzung des Reaktionsprodukts aus Cyanurchlorid und (II) mit (IV) in Gegenwart des Amins (VI) als Säurefänger durchführt.

Gegenstand der Erfindung ist also ein Verfahren zur Herstellung von Verbindungen der Formel (I) durch Umsetzung von Cyanurchlorid mit einem 4,4'-Diaminostilben-2,2'-disulfonsäuresalz der Formel (II) im Molverhältnis 2:1, anschließende Umsetzung der erhaltenen Verbindung der Formel 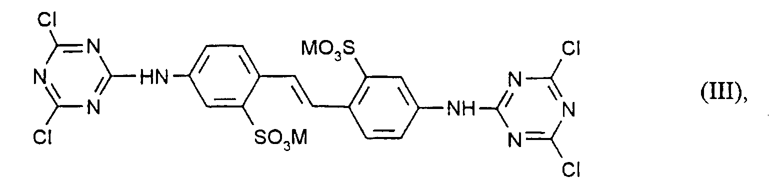 worin M die oben angegebene Bedeutung besitzt,
mit 2 Äquivalenten eines Anilins der Formel 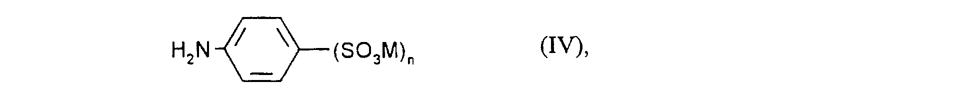 worin M und n die oben angegebene Bedeutung besitzt,
und anschließende Umsetzung der erhaltenen Verbindung der Formel 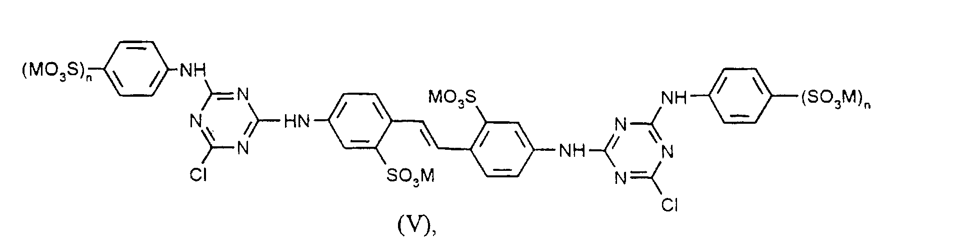 worin M und n die oben angegebene Bedeutung besitzen
mit 2 Äquivalenten eines Amins der Formel XH (VI),
worin X die oben angegebene Bedeutung besitzt,
dadurch gekennzeichnet, daß man (III) mit (IV) in Gegenwart des Amins (VI) als Säurefänger umsetzt und zu diesem Zweck Amin (VI) so zudosiert, daß der pH-Wert im Bereich von 3,0 bis 7,0, vorzugsweise im Bereich von 3,5 bis 6,0, insbesondere im Bereich von 4,0 bis 6,0 gehalten wird, und für die Umsetzung von (V) das Amin (VI) aus seiner protonierten Form durch eine geeignete Base befreit.

Das erfindungsgemäße Verfahren führt zu einer deutlichen Erhöhung der Reaktionsgeschwindigkeit, was zu einer Umsetzung bei erheblich tieferen Temperaturen (d.h. unter milderen Bedingungen) als bisher ohne Umsatzeinbußen ausgenutzt werden kann.

Bei der Durchführung des erfindungsgemäßen Verfahrens wird im allgemeinen in der ersten Stufe ein 4,4'-Diaminostilben-2,2'-disulfonsäuredialkalisalz der Formel (II) in Form einer wäßrigen Lösung einer wäßrigen Cyanurchlorid-Suspension im Temperaturbereich von 0 bis 25°C und im pH-Bereich von 3,5 bis 5,5 in Anwesenheit oder unter gleichzeitiger Zugabe eines wasserlöslichen, säurebindenden Mittels zur Neutralisation der freigesetzten Salzsäure zugegeben. Zu diesem Zweck geeignete wasserlösliche säurebindende Mittel sind beispielsweise Alkalihydroxide, Alkalicarbonate und Alkalihydrogencarbonate wie Natriumhydroxid, Kaliumhydroxid, Natriumcarbonat, Kaliumcarbonat, Natriumhydrogencarbonat und Kaliumhydrogencarbonat oder Triethanolamin. Diese können in fester oder flüssiger Form, z.B. nach und nach über eine Förderschnecke, oder vorteilhaft auch in Form ihrer wäßrigen Lösungen eingesetzt werden.

Die wäßrige Cyanurchlorid-Suspension enthält üblicherweise 0,05 - 2 Gew.-% (bezogen auf Cyanurchlorid) benetzendes Tensid, vorzugsweise aus der Reihe alkoxylierter Fettalkohole, insbesondere mindestens einen mit 3 bis 10 Mol Ethylenoxid und 0 bis 25 Mol Propylenoxid alkoxylierten C₈-C₁₄-Fettalkohol, z.B. den Polyether aus Laurylalkohol und 5 Mol Ethylenoxid und/oder den Polyether aus einem C₁₀-Alkohol und 6 Mol Ethylenoxid und 8 Mol Propylenoxid.

Weiterhin kann die Cyanurchlorid-Suspension auch 0,05 bis 2,0 Gew.% (bezogen auf Cyanurchlorid) Entschäumer enthalten, der z.B. etwa zur Hälfte aus einer Mischung von C₁₅-Alkansulfamid und Ammoniumsalz der C₁₅-Alkansulfonsäure und zur anderen Hälfte aus dem entsprechenden C₁₅-Alkan besteht.

Eine bevorzugte Ausführungsform des erfindungsgemäßen Verfahrens besteht darin, die Zugabe der Lösung von (II), die zusätzlich eine zur Neutralisation der bei der Reaktion entstehenden Salzsäure gerade ausreichende Menge eines wasserlöslichen säurebindenden Mittels enthält, zur wäßrigen Cyanurchlorid-Suspension pH-gesteuert bei einem pH-Sollwert aus dem Bereich 3,5 - 5,5, vorzugsweise 4,0 - 5,0, bei einer Temperatur von 5 bis 20°C durchzuführen, wobei die Abweichung des Sollwertes in der Regel nicht mehr als -0,5 oder + 0,3 pH-Einheiten beträgt.

Praktisch ist der Endpunkt für die Zugabe der Lösung von (II) dann erreicht, wenn in 10 Minuten weniger als 0,5 % Lösung von (II) verbraucht werden.

Die zur Neutralisation der bei der Reaktion entstehenden Salzsäure gerade ausreichende Menge eines wasserlöslichen säurebindenden Mittels beträgt theoretisch 2 Äquivalente.

Die Reaktion von (III) mit (IV) in Gegenwart des Amins (VI) kann in wäßriger Phase bei Temperaturen von vorzugsweise 15 bis 75°C, insbesondere bei 20 bis 60°C, ganz besonders bevorzugt bei 20 bis 40°C durchgeführt werden. Nach einer besonderen Ausführungsform wird das Amin (VI), gegebenenfalls in Form einer Lösung, in die Reaktionsmischung, die (III) und (IV) enthält, pH-gesteuert eintitriert. Bei einer solchen pH-gesteuerten Zugabe von (VI) dient ein aus dem pH-Bereich zwischen 3 und 7 ausgewählter pH-Wert als Steuergröße. Wird dieser Sollwert unterschritten, fließt solange (VI) in die gerührte Lösung oder Suspension von (III) ein, bis der ursprüngliche Wert wieder erreicht ist. Das kann manuell oder vorzugsweise automatisch mit Hilfe eines Titrators erfolgen. Als Titrator eignet sich beispielsweise ein DULCOMETER® vom Typ PR F2K2 der Firma Pro Minent.

Die Abweichung vom pH-Sollwert beträgt in der Regel nicht mehr als -0,5 oder +0,3 pH-Wert-Einheiten. Wenn (III) verbraucht ist und somit unter diesen Bedingungen keine weitere Salzsäure mehr entsteht, stellt sich der als Sollwert gewählte pH wieder ein, ohne noch einmal wieder abzusinken, und der Zufluß stoppt.

Praktisch ist der Endpunkt für die Zugabe von (VI) bereits dann erreicht, wenn in 10 Minuten weniger als 0,5 % von (VI) verbraucht werden.

Besonders bevorzugt ist die pH-gesteuerte Zugabe von (VI) bei einem pH-Sollwert aus dem Bereich von 4,0 bis 6,0, wobei auch hier die Abweichung von diesem Sollwert in der Regel nicht mehr als -0,5 oder +0,3 pH-Einheiten beträgt.

Die Angaben des Molverhältnisses von 2:1 bei der Umsetzung von (III) und (IV) und von 2 Äquivalenten (VI) bei der Umsetzung mit (V) sollen selbstverständlich nur die theoretische Stöchiometrie der Umsetzung kennzeichnen. Das schließt also nicht aus, daß z.B. (IV) im bis zu 10 %igen Überschuß oder im bis zu 10 %igen Unterschuß und z.B. (VI) zur Vervollständigung der Reaktion vorteilhaft auch im 5 bis 30 %igen Überschuß eingesetzt wird.

Bevorzugt läßt man (IV) als wäßrige 40 bis 80°C heiße Lösung mit einer Konzentration über 1,5 mol/l zur Suspension von ( III ) zulaufen.

Bevorzugte Amine der Formel (VI) sind Methylamin, Ethylamin, Dimethylamin, Diethylamin, Morpholin, 2-Hydroxyethylamin, 2-Hydroxypropylamin, insbesondere Di-(2-hydroxyethyl)-amin und Di-(2-hydroxypropyl)-amin.

Das Amin (VI) wird durch den bei der Reaktion von (III) mit (IV) freigesetzten Chlorwasserstoff mit hoher Selektivität protoniert und wirkt in dieser Form nicht als Reaktionspartner für (III). Das Amin (IV) oder dessen wäßrige Lösung kann bereits die zur Neutralisation erforderliche Menge (VI) enthalten. In diesem Fall kann man die Titration beenden, wenn innerhalb von 10 Minuten weniger als 0,5 % der theoretisch erforderlichen Gesamtmenge (VI) verbraucht werden.

Im Anschluß an die Reaktion zu (V) wird durch Zugabe von vorzugsweise je 2 Äquivalenten (pro Amin) eines Säurefängers das protonierte Amin zuerst zum Amin (VI) deprotoniert und die bei der Reaktion von (V) mit dem Amin (VI) entstehende Salzsäure abgefangen. Die Umsetzung von (V) und (VI) kann in bekannter Weise z.B. durch 2- bis 4-stündiges Erhitzen der Reaktionslösung auf Temperaturen von 90°C bis Siedetemperatur in Gegenwart eines Alkalihydroxids, eines substituierten Amins oder vorzugsweise eines Alkalicarbonats oder -hydrogencarbonats als Säurefänger durchgeführt werden. Bevorzugt erfolgt die Umesterung von (V) und (VI) in einem pH-Bereich unter 10,0, wobei man den Säurefänger pH gesteuert eintitriert.

Die Aufarbeitung der nach dem erfindungsgemäßen Verfahren hergestellten Verbindungen (I) kann z.B. durch Aussalzen und Abfiltrieren erfolgen, oder man fällt (I) als in Wasser schwerlösliche freie Säure (M = H) aus der warmen Lösung aus und filtriert sie ab. Der feuchte Kristallkuchen kann dann in bekannter Weise zur Herstellung einer lagerstabilen flüssigen Zubereitung gegebenenfalls unter Zusatz eines oder mehrerer Formulierhilfsmittel, wie nichtionische oder anionische Tenside und/oder polare organische Lösungsvermittler wie Polyglykole oder Harnstoff, in Wasser gelöst werden, wobei man im Falle der freien Säure soviel Base zusetzt, daß diese wieder in ein gut wasserlösliches Salz zurückverwandelt wird. Eine sehr einfache, kostengünstige Aufarbeitung besteht darin, die Rohlösung von (I) durch Membrantrennverfahren unter Druck (Druckpermeation) weitgehend zu entsalzen, aufzukonzentrieren und direkt in die handelsübliche stabile flüssige Zubereitung zu überführen, z.B. nach DE-PS 32 34 784 oder DE-OS 22 04 725.

Diese rationelle Aufarbeitungsmethode läßt sich um so erfolgreicher anwenden, je reiner die Aufheller-Rohlösungen sind, die das Verfahren liefert, da die Nebenprodukte und Verunreinigungen bei der Druckpermeation mit Ausnahme der niedermolekularen Anteile nicht mit dem Permeat ausgeschleust werden, somit letztlich im Aufhellerpräparat verbleiben und dessen Effizienz beeinträchtigen können.

Die Prozentangaben der nachfolgenden Beispiele beziehen sich jeweils auf das Gewicht.

### Beispiel 1

Im Reaktor werden 700 ml Wasser und 10 g Kochsalz eingetragen und 10 min verrührt. Anschließend setzt man unter Rühren 1,0 g eines Polyethers aus Isodecylalkohol, 6 mol Ethylenoxid und 8 mol Propylenoxid zu und kühlt den Ansatz auf ca. 10 °C ab.

Man trägt unter Rühren 100 g Cyanurchlorid (0,542 mol) ein und spült mit 100 ml Wasser nach und verrührt die Suspension, bis der pH von selbst auf 4,5 gefallen ist.

Man verwendet einen automatischen Titrator, stellt diesen auf den oberen Grenzwert von pH 4,5 ein und titriert eine wäßrige auf 10°C gekühlte Lösung ein, die pro Liter 0,25 mol 4,4'-Diaminostilben-2,2'-disulfonsäure-dinatriumsalz und 0,25 mol Natriumcarbonat enthält, wobei man die Temperatur im Ansatz auf 18°C ansteigen läßt.

Theoretisch können 1 084 ml Lösung verbraucht werden. Der Endpunkt der Reaktion ist erreicht, wenn innerhalb von 10 Minuten weniger als 5 ml verbraucht werden. Das ist nach 2 bis 2,5 Stunden bei einem Verbrauch von 99 % der Theorie der Fall. Es entsteht eine gut rührbare hellgelbe Suspension.

Man wechselt die Titratorvorlage. Die Titratorvorlage enthält 87,2 g 80 %ige wäßrige Diethanolaminlösung (0,664 mol). Man rührt 30 min bei pH 4,5 nach.

Anschließend läßt man 500 ml Natriumsulfanilatlösung, die in diesen 500 ml 0,528 mol Natriumsulfanilat enthält, innerhalb 30 Minuten einlaufen, wobei man die Temperatur im Reaktor auf 55°C erhöht. Man rührt 1 Stunde bei 55°C nach. Bis zu diesem Zeitpunkt werden über den Titrator ca. 74 g 80%ige wäßrige Diethanolaminlösung (0,56 mol) eindosiert. Die Aufnahme beträgt am Ende der Nachrührzeit weniger als 2 ml in 10 Minuten.

Man läßt den Rest der wäßrigen Diethanolaminlösung zulaufen und anschließend innerhalb 30 Minuten 402 g 15 %ige wäßrige Sodalösung (0,569 mol).

Das Gemisch wird innerhalb einer Stunde auf 101°C erwärmt und 2 Stunden bei dieser Temperatur gehalten.

Man erhält 3,1 kg einer wäßrigen Rohlösung mit einer spezifischen Extinktion von 54 bei 350 nm. Die Rohlösung enthält 3,5 % (0,11 kg) Kochsalz und 10 % (0,31 kg) Aktivsubstanz, die im wesentlichen der Verbindung der Formel 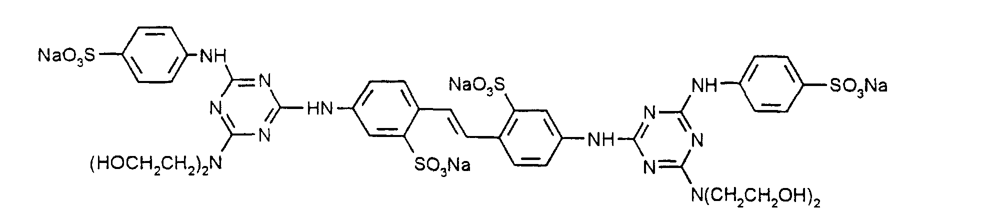 entspricht.

Die Rohlösung wird in der in DE-C 32 34 784, Beispiel 2 angegebenen Weise durch Druckpermeation bis auf einen Restgehalt von 0,5 % Kochsalz entsalzt und dann auf ein Gewicht von 1,34 kg aufkonzentriert. Man erhält eine wäßrige Zubereitung mit einer spezifischen Extinktion von 125 bei 350 nm, 23 % Aktivsubstanz und einem Elektrolytgehalt von <0,5 %.

Zum gleichen Ergebnis kommt man, wenn man die 500 ml Natriumsulfanilatlösung, die in diesen 500 ml 0,528 mol Natriumsulfanilat enthält, analog der obigen Verfahrensweise zugibt, wobei die Temperatur im Reaktor zunächst auf 30 bis 35°C und nach 90 Minuten auf 65°C erhöht wird. Anschließend rührt man 15 Minuten bei 65°C nach und verfährt wie oben beschrieben.

### Beispiel 2

Man wiederholt Beispiel 1 mit dem Unterschied, daß man 300 ml Natriumsulfanilatlösung einer 60°C heißen Lösung einsetzt, die 0,528 mol Natriumsulfanilat enthält. Man erhält 2,9 kg einer wäßrigen Rohlösung mit einer spezifischen Extinktion von 58 bei 350 nm. Die Aufarbeitung erfolgt analog Beispiel 1.

### Beispiel 3

Man wiederholt Beispiel 1 mit dem Unterschied, daß man anstelle von Di-(2-hydroxyethyl)-amin eine äquivalente Menge (0,664 mol) Di-(2-hydroxypropyl)-amin einsetzt. Die erhaltene wäßrige Rohlösung (3,1 kg) wird durch Druckpermeation bis auf einen Restgehalt von 0,5 % NaCl entsalzt und dann aufkonzentriert, bis die spezifische Extinktion 142 beträgt. Die erhaltenen 1,17 kg Konzentrat werden mit 130 g Octaethylenglykol-Homologengemisch (durchschnittliches Molekulargewicht 400) verrührt. Man erhält 1,3 kg einer stabilen wäßrigen Zubereitung mit einer spezifischen Extinktion von 128 und einem Elektrolytgehalt <0,5%.

Der Aufheller entspricht der Formel

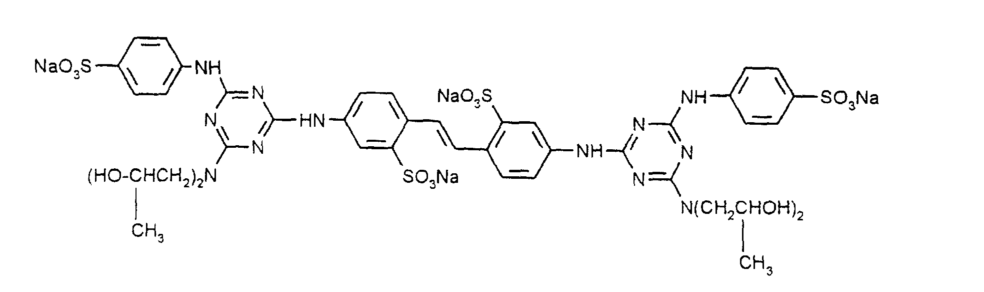

### Beispiel 4

In den Reaktor werden 500 ml Wasser und 10 g Kochsalz eingetragen und 10 min verrührt. Anschließend setzt man unter Rühren 1,0 g eines Polyethers aus Isodecylalkohol, 6 mol Ethylenoxyd und 8 mol Propylenoxyd zu und kühlt den Ansatz auf ca. 10 °C ab.

Man trägt unter Rühren 100 g Cyanurchlorid (0,542 mol) ein und spült mit 100 ml Wasser nach und verrührt die Suspension, bis der pH von selbst auf 4,3 gefallen ist.

Man verwendet einen automatischen Titrator, stellt diesen auf den oberen Grenzwert von pH 4,3 ein und titriert eine wäßrige auf 10°C gekühlte Lösung ein, die pro Liter 0,25 mol 4,4'-Diaminostilben-2,2'-disulfonsäure-dinatriumsalz und 0,25 mol Natriumcarbonat enthält, wobei man die Temperatur der Reaktionsmischung auf 15°C ansteigen läßt.

Theoretisch können 1 084 ml Lösung verbraucht werden. Der Endpunkt der Reaktion ist erreicht, wenn innerhalb von 10 Minuten weniger als 5 ml verbraucht werden. Das ist nach 2 bis 2,5 Stunden bei einem Verbrauch von 99 % der Theorie der Fall. Es entsteht eine gut rührbare hellgelbe Suspension.

Man wechselt die Titratorvorlage. Die Titratorvorlage enthält in 700 ml wäßrige Lösung von 0,61 mol Natriumsulfanilat und 0,64 mol Diethanolamin. Man erhöht die Temperatur im Reaktor auf 55°C.

Theoretisch sollten 623 ml Lösung verbraucht werden. Der Endpunkt der Reaktion ist erreicht, wenn innerhalb von 10 Minuten weniger als 3 ml verbraucht werden. Das ist nach 1 Stunde bei einem Verbrauch von 101% der Theorie der Fall.

Man läßt 17,2 g 50%ige wäßrige Diethanolaminlösung zulaufen und gibt anschließend innerhalb 30 Minuten 96 g Natriumhydrogencarbonat (1,14 mol) zu.

Das Gemisch wird innerhalb einer Stunde auf 101°C erwärmt und 3 Stunden bei dieser Temperatur gehalten.

Man erhält 2,7 kg einer wäßrigen Rohlösung mit einer spezifischen Extinktion von 62 bei 350 nm. Die Rohlösung enthält 4 % (0,11 kg) Kochsalz und 11,5 % (0,31 kg) Aktivsubstanz, die im wesentlichen der Verbindung der Formel 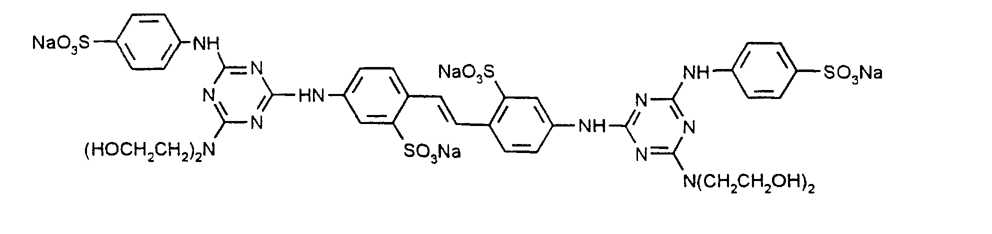 entspricht.

Die Rohlösung wird in der in DE-C 32 34 784, Beispiel 2 angegebenen Weise durch Druckpermeation bis auf einen Restgehalt von 0,5 % Kochsalz entsalzt und dann auf ein Gewicht von 1,34 kg aufkonzentriert. Man erhält eine wäßrige Zubereitung mit einer spezifischen Extinktion von 125 bei 350 nm, 23 % Aktivsubstanz und einem Elektrolytgehalt von <0,5 %.

### Beispiel 5

Im Reaktor werden 700 ml Edelwasser und 10 g Kochsalz eingetragen und 10 min verrührt. Anschließend setzt man unter Rühren 1,0 g eines Polyethers aus Isodecylalkohol, 6 mol Ethylenoxyd und 8 mol Propylenoxyd zu und kühlt den Ansatz auf 10°C ab.

Man trägt unter Rühren 100 g Cyanurchlorid (0,542 mol) ein und spült mit 100 ml Edelwasser nach und verrührt die Suspension, bis der pH auf 4,5 gefallen ist.

Man verwendet einen automatischen Titrator, stellt diesen auf den oberen Grenzwert von pH 4,5 ein und titriert eine wäßrige auf 10°C-gekühlte Lösung ein, die in 1 200 ml 0,3 mol 4,4'-Diaminostilben-2,2'-disulfonsäure-dinatriumsalz und 0,3 mol Natriumcarbonat enthält, wobei man die Temperatur im Ansatz auf 18°C ansteigen läßt.

Theoretisch können 1 084 ml verbraucht werden. Der Endpunkt der Reaktion ist erreicht, wenn innerhalb von 10 Minuten weniger als 5 ml verbraucht werden. Das ist nach 2 bis 2,5 Stunden bei einem Verbrauch von 99 % der Theorie der Fall. Es entsteht eine gutrührbare hellgelbe Suspension.

Man wechselt die Titratorvorlage. Die Titratorvorlage enthält 87,2 g 80 %ige wäßrige Diethanolaminlösung (0,664 mol). Man rührt 30 min bei pH 5,5 nach.

Anschließend läßt man 50,2 g Anilin (0,54 mol) innerhalb 30 Minuten einlaufen, wobei man die Temperatur im Reaktor auf 20°C hält. Man rührt 1 Stunde bei 20°C nach. Bis zu diesem Zeitpunkt werden über den Titrator ca. 74 g 80%ige wäßrige Diethanolaminlösung (0,56 mol) eindosiert. Die Aufnahme beträgt am Ende der Nachrührzeit weniger als 2 ml in 10 Minuten. Man läßt bei 20°C zuerst innerhalb von 30 min 216 g Natronlauge 10 %ig (0,54 mol) und dann den Rest der wäßrigen Diethanolaminlösung zulaufen.

Man erhitzt den Ansatz auf 95°C und hält schon während der Aufheizphase durch titrimetrische Zugabe von Natronlauge (10 %ig) den pH-Wert auf 7,5. Man hält pH und Temperatur 4 Stunden konstant.

Man läßt auf 85°C abkühlen und stellt bei dieser Temperatur durch Zugabe von Salzsäure einen pH von 4,2 ein. Man rührt 30 min nach und läßt dabei auf 50 bis 55°C abkühlen.

Der Ansatz wird über eine Nutsche abgesaugt und sorgfältig mit Wasser gewaschen. Nach dem Trocknen bei 50°C im Vakuum erhält man 245 g Produkt.

Das Produkt entspricht der Verbindung der Formel

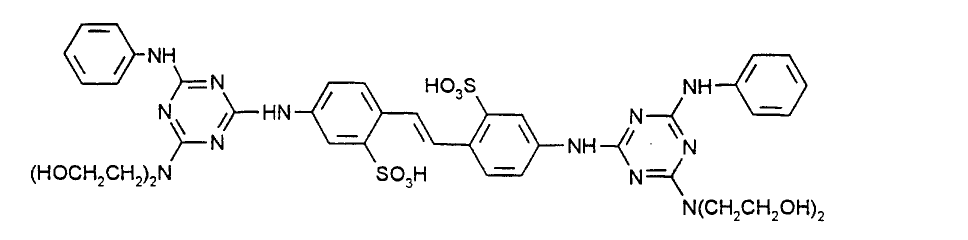

## Patentansprüche

1. Verfahren zur Herstellung von Verbindungen der Formel 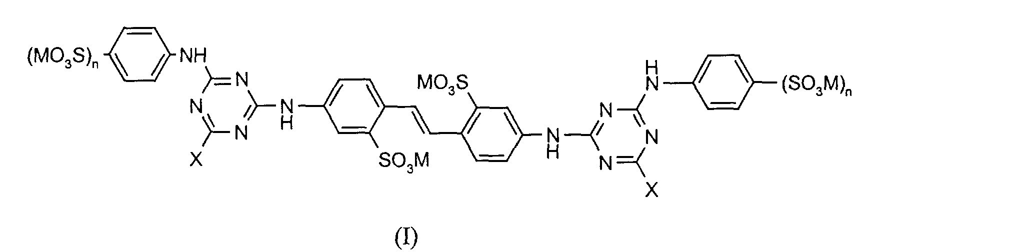 worin
n für 0 oder 1 steht,
M für ein Alkalimetallion oder ein gegebenenfalls substituiertes Ammoniumion und
X für N-Alkylamino oder N,N-Dialkylamino stehen, wobei unter Alkylresten in den zusammengesetzten Begriffen N-Alkylamino und N,N-Dialkylamino solche mit bis zu 4 C-Atomen verstanden werden, die gegebenenfalls durch ein O-Atom unterbrochen sind und/oder Hydroxy, Carbamoyl, Cyano oder Sulfo als Substituenten tragen können, wobei im Fall von N,N-Dialkylamino die beiden gegebenenfalls durch ein Heteroatom aus der Reihe O, N, S, unterbrochenen Alkylreste gemeinsam mit dem N-Atom, an das sie gebunden sind, einen gesättigten 5- oder 6-gliedrigen Heterocyclus bilden können,
durch Umsetzung von Cyanurchlorid mit einem 4,4'-Diaminostilben-2,2'-disulfonsäuresalz der Formel 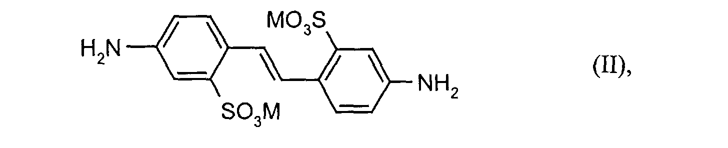 worin M die oben angegebene Bedeutung hat,
im Molverhältnis 2:1, anschließende Umsetzung der erhaltenen Verbindung der Formel 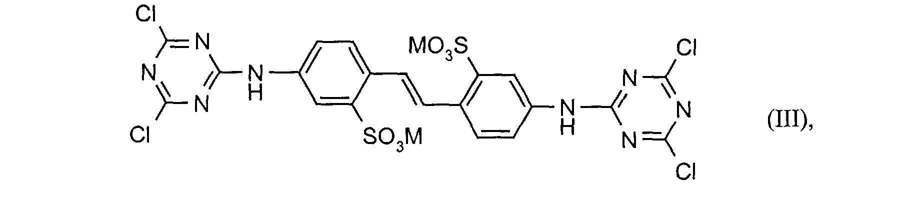 worin M die oben angegebene Bedeutung besitzt,
mit 2 Äquivalenten eines Anilins der Formel 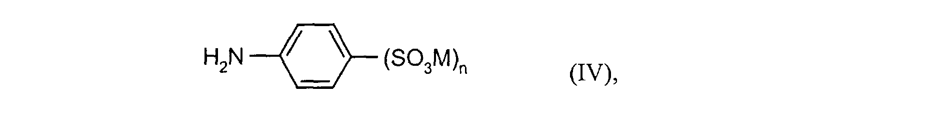 worin M und n die oben angegebene Bedeutung hat,
und anschließende Umsetzung der erhaltenen Verbindung der Formel 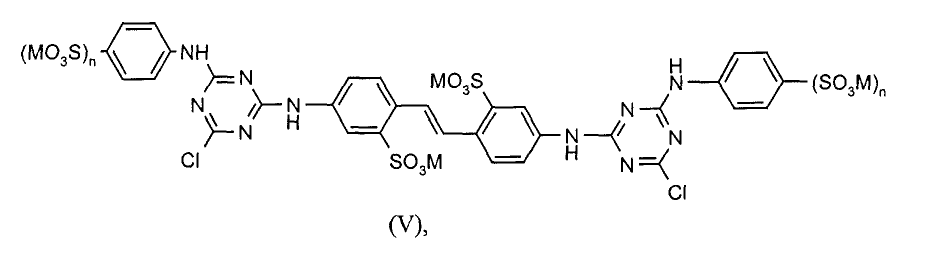 worin M und n die oben angegebene Bedeutung besitzt,
mit 2 Äquivalenten eines Amins der allgemeinen Formel XH (VI),
worin X die oben angegebene Bedeutung besitzt,
**dadurch gekennzeichnet, dass** man (III) mit (IV) in Gegenwart des Amins (VI) als Säurefänger umsetzt und zu diesem Zweck Amin (VI) so zudosiert, dass der pH-Wert im Bereich von 3,0 bis 7,0 gehalten wird, und für die Umsetzung von (V) das Amin (VI) aus seiner protonierten Form durch eine geeignete Base befreit.

2. Verfahren nach Anspruch 1, **dadurch gekennzeichnet, dass** die Zugabe von (VI) im Temperaturbereich von 15 bis 75°C bei einem pH-Sollwert von 3,5 bis 6,0 erfolgt und die Abweichung von diesem Sollwert nicht mehr als -0,5 bis +0,3 pH-Einheiten beträgt.

3. Verfahren nach Anspruch 1, **dadurch gekennzeichnet, dass** die Zugabe von (VI) im Temperaturbereich von 20 bis 60°C bei einem pH-Sollwert von 4,0 bis 6,0 erfolgt und die Abweichung von diesem Sollwert nicht mehr als -0,5 bis +0,3 pH-Einheiten beträgt.

4. Verfahren nach Anspruch 1, **dadurch gekennzeichnet, dass** die Umsetzung von Cyanurchlorid mit der Verbindung (II) in der Weise durchgeführt wird, dass eine wäßrige Lösung von (II), die zusätzlich eine zur Neutralisation der bei der Reaktion entstehenden Salzsäure gerade ausreichende Menge eines wasserlöslichen säurebindenden Mittels enthält, pH-gesteuert bei einem pH-Sollwert aus dem Bereich 3,5 bis 5,5 und einer Temperatur von 5 bis 20°C der wäßrigen Cyanurchlorid-Suspension zugegeben wird.

5. Verfahren nach Anspruch 1 und 4, **dadurch gekennzeichnet, dass** die Umsetzung von Cyanurchlorid mit der Verbindung der Formel (II) in der Weise durchgeführt wird, dass die wäßrige Lösung von (II) auf 5 bis 15°C gekühlt eingesetzt wird.

6. Verfahren nach Anspruch 1 und 4, **dadurch gekennzeichnet, dass** die Verbindung (IV) als wäßrige 40 bis 80°C heiße Lösung mit einer Konzentration größer 1,5 mol/Liter eingesetzt wird.

7. Verfahren nach Anspruch 1, **dadurch gekennzeichnet, dass** anschließend der isolierte feuchte Kristallkuchen gegebenenfalls unter Zusatz eines oder mehrerer Formulierhilfsmittel in Wasser gelöst wird und die Rohlösung der Verbindung (I) mit Hilfe eines Membranverfahrens unter Druck entsalzt und aufkonzentriert.

8. Verfahren nach Anspruch 1 und 4, **dadurch gekennzeichnet, dass** in der Verbindung (I) X für Di-(2-hydroxyethyl)-amino steht.

9. Verfahren nach Anspruch 1, **dadurch gekennzeichnet, dass** (IV) im bis zu 10 %igen Überschuß oder im bis zu 10 %igen Unterschuß und ( VI ) im 5 bis 30 %igen Überschuß eingesetzt wird.

10. Verfahren nach Anspruch 1 **dadurch gekennzeichnet, dass** die gegebenenfalls wäßrige Lösung von (IV) die zur Neutralisation des bei der Reaktion entstehenden Chlorwasserstoffs erforderliche Menge (VI) enthält und man mit dieser Kombination titriert, bis in 10 Minuten weniger als 0,5% der theoretisch erforderlichen Gesamtmenge verbraucht werden.

## Claims

1. Process for the preparation of compounds of the formula 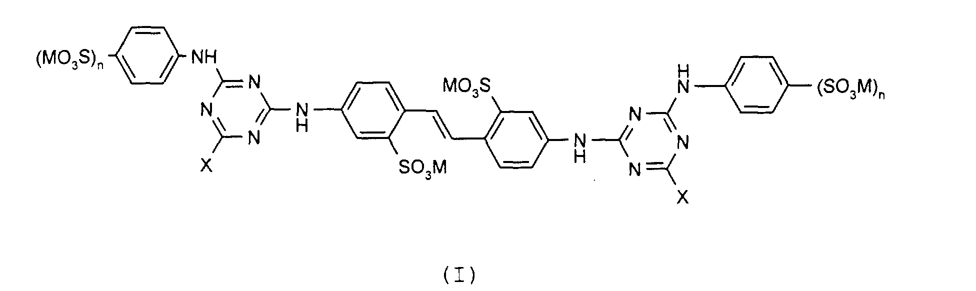 in which
n is 0 or 1,
M is an alkali metal ion or an optionally substituted ammonium ion and
X is N-alkylamino or N,N-dialkylamino, where the alkyl radicals in the combined terms N-alkylamino and N,N-dialkylamino are to be understood as meaning those having up to 4 carbon atoms, which may be interrupted by an O atom and/or may carry, as substituent, hydroxyl, carbamoyl, cyano or sulpho, and when it is N,N-dialkylamino, the two alkyl radicals which are optionally interrupted by a heteroatom from the series O, N and S, together with the N-atom to which they are bonded may form a saturated 5- or 6-membered heterocycle,
by reaction of cyanuric chloride with a 4,4'-diaminostilbene-2,2'-disulphonic acid salt of the formula 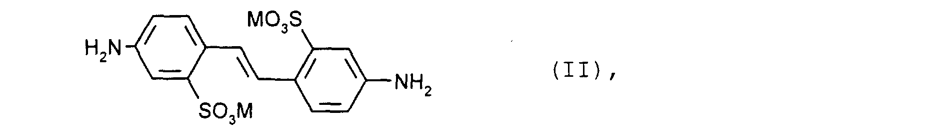 in which M is as defined above,
in the molar ratio 2:1, subsequent reaction of the resulting compound of the formula 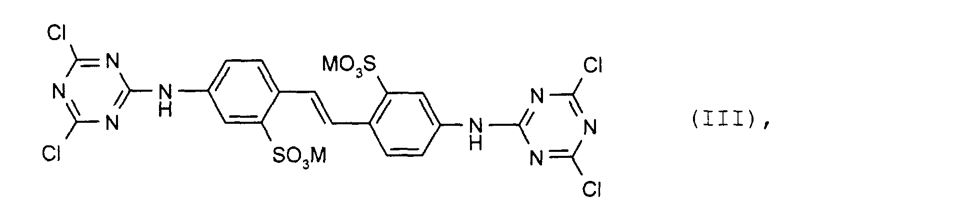 in which M is as defined above,
with 2 equivalents of an aniline of the formula 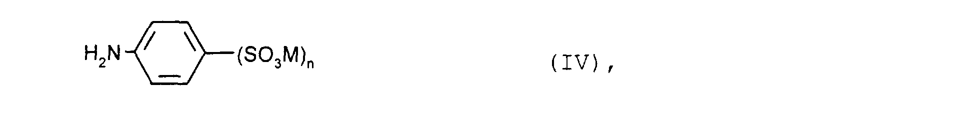 in which M and n are as defined above,
and subsequent reaction of the resulting compound of the formula 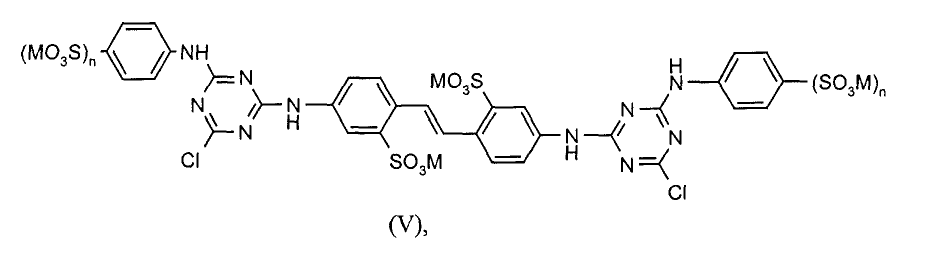 in which M and n are as defined above,
with 2 equivalents of an amine of the general formula XH (VI),
in which X is as defined above,
**characterized in that** (III) is reacted with (IV) in the presence of the amine (VI) as acid-absorber and that, for this purpose, amine (VI) is metered in at a rate such that the pH is maintained in the range from 3.0 to 7.0, and that for the reaction of (V), the amine (VI) is freed from its protonated form by a suitable base.

2. Process according to Claim 1, **characterized in that** the addition of (VI) takes place in the temperature range from 15 to 75°C at a set pH of from 3.5 to 6.0 and that the deviation from this set value is no more than from -0.5 to +0.3 pH units.

3. Process according to Claim 1, **characterized in that** the addition of (VI) takes place in the temperature range from 20 to 60°C at a set pH of from 4.0 to 6.0 and the deviation from this set value is no more than from -0.5 to +0.3 pH units.

4. Process according to Claim 1, **characterized in that** the reaction of cyanuric chloride with the compound (II) is carried out by adding an aqueous solution of (II), which additionally comprises an amount of a water-soluble acid-binding agent just sufficient to neutralize the hydrochloric acid formed during the reaction, to the aqueous cyanuric chloride suspension under pH control at a set pH from the range 3.5 to 5.5 and a temperature from 5 to 20°C.

5. Process according to Claim 1 and 4, **characterized in that** the reaction of cyanuric chloride with the compound of the formula (II) is carried out in a manner which involves the aqueous solution of (II) used being cooled to from 5 to 15°C.

6. Process according to Claim 1 and 4, **characterized in that** the compound (IV) used is an aqueous solution having a temperature of from 40 to 80°C and a concentration greater than 1.5 mol/litre.

7. Process according to Claim 1, **characterized in that** the isolated moist crystal cake is then dissolved in water, optionally with the addition of one or more formulating auxiliaries, and the crude solution of the compound (I) is desalinated using a membrane method under pressure and concentrated.

8. Process according to Claim 1 or 4, **characterized in that** in the compound (I), X is di-(2-hydroxyethyl)-amino.

9. Process according to Claim 1, **characterized in that** (IV) is used in up to 10% excess or in up to 10% deficit, and ( VI ) is used in from 5 to 30% excess.

10. Process according to Claim 1, **characterized in that** the optionally aqueous solution of (IV) comprises the amount of (VI) required to neutralize the hydrogen chloride produced during the reaction, and this combination is titrated until in 10 minutes, less than 0.5% of the total amount theoretically required is consumed.

## Revendications

1. Procédé pour la préparation de composés de formule 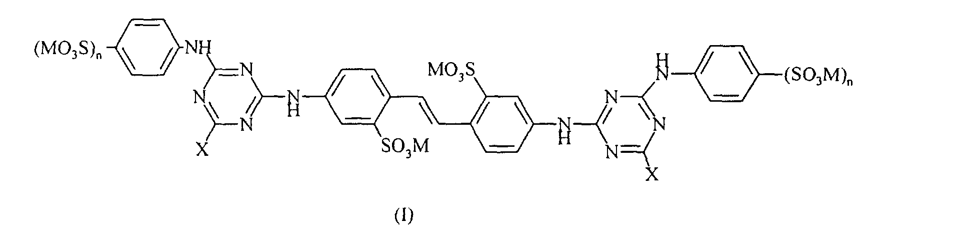 dans laquelle
n est égal à 0 ou 1,
M représente un ion de métal alcalin ou un ion ammonium éventuellement substitué et
X représente un groupe N-alkylamino ou N,N-dialkylamino dont les groupes alkyle contiennent jusqu'à 4 atomes de carbone, sont éventuellement interrompus par un atome d'oxygène et/ou peuvent porter des substituants hydroxy, carbamoyle, cyano ou sulfo, et dans le cas du groupe N,N-dialkylamino, les deux groupes alkyle, éventuellement interrompus par un hétéroatome choisi parmi O, N et S, peuvent former avec l'atome d'azote auquel ils sont reliés un hétérocycle saturé à 5 ou 6 chaînons,
par réaction du chlorure de cyanuryle avec un sel de l'acide 4,4'-diaminostilbène-2,2'-disulfonique de formule 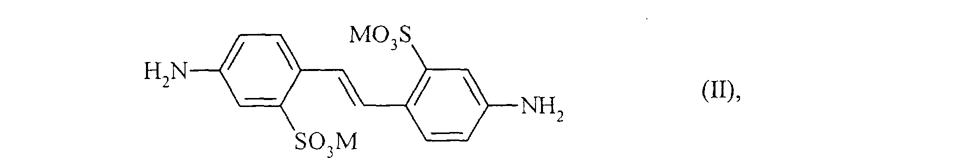 dans laquelle M a les significations indiquées ci-dessus,
au rapport molaire de 2:1, la réaction donnant le composé de formule 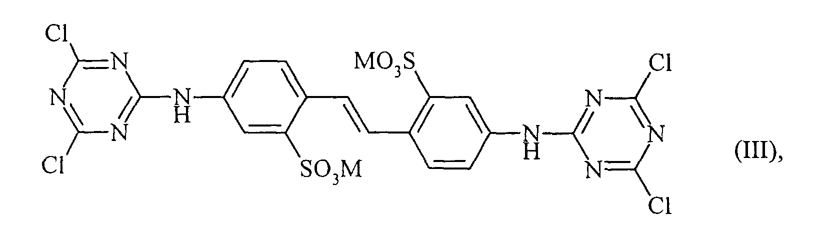 dans laquelle M a les significations indiquées ci-dessus,
qu'on fait réagir avec 2 équivalents d'une aniline de formule 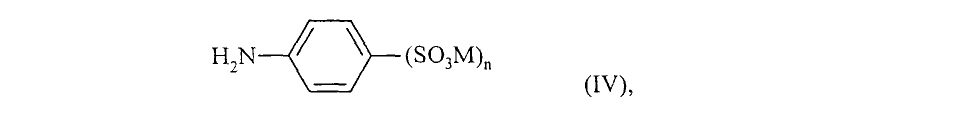 dans laquelle M et n ont les significations indiquées ci-dessus,
la réaction donnant le composé de formule 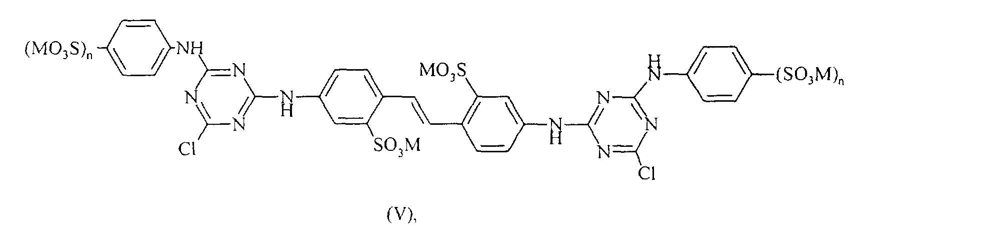 dans laquelle M et n ont les significations indiquées ci-dessus,
qu'on fait réagir avec 2 équivalents d'une amine de formule générale XH (VI),
dans laquelle X a les significations indiquées ci-dessus,
ce procédé se caractérisant en ce que l'on fait réagir (III) avec (IV) en présence de l'amine (VI) qui sert de capteur d'acide et on ajoute à cet effet l'amine (VI) en quantité suffisante pour maintenir le pH dans l'intervalle de 3,0 à 7,0, et pour la réaction avec (V), on libère l'amine (VI) de sa forme protonisée à l'aide d'une base appropriée.

2. Procédé selon la revendication 1, **caractérisé en ce que** (VI) est ajoutée dans l'intervalle de température de 15 à 75°C en quantité suffisante pour maintenir un pH qui se situera entre 3,5 et 6,0 et dont on s'écartera au maximum de -0,5 à +0,3 unité de pH.

3. Procédé selon la revendication 1, **caractérisé en ce que** (VI) est ajoutée dans l'intervalle de température de 20 à 60°C en quantité suffisante pour maintenir un pH qui se situera entre 4,0 et 6,0 et dont on s'écartera au maximum de -0,5 à +0,3 unité de pH.

4. Procédé selon la revendication 1, **caractérisé en ce que** la réaction du chlorure de cyanuryle avec le composé (II) est réalisée par addition à la suspension aqueuse du chlorure de cyanuryle d'une solution aqueuse de (II) contenant en outre une quantité d'un capteur d'acide soluble dans l'eau juste suffisante pour neutraliser l'acide chlorhydrique formé dans la réaction, avec contrôle du pH et en maintenant un pH qui se situe dans l'intervalle de 3,5 à 5,5, à une température de 5 à 20°C.

5. Procédé selon les revendications 1 et 4, **caractérisé en ce que** la réaction du chlorure de cyanuryle avec le composé de formule (II) est réalisée avec la solution aqueuse de (II) refroidie à une température de 5 à 15°C.

6. Procédé selon les revendications 1 et 4, **caractérisé en ce que** le composé (IV) est mis en oeuvre à l'état de solution aqueuse chauffée à une température de 40 à 80°C et à une concentration supérieure à 1,5 mol/l.

7. Procédé selon la revendication 1, **caractérisé en ce que** l'on dissout ensuite dans l'eau le gâteau de cristaux humide isolé, éventuellement avec adjonction d'un ou plusieurs produits auxiliaires de formulation et on soumet la solution brute du composé (I) à déminéralisation et concentration par un procédé à membrane sous pression.

8. Procédé selon les revendications 1 et 4, **caractérisé en ce que**, dans la formule du composé (I), X représente un groupe di-(2-hydroxyéthyl)-amino.

9. Procédé selon la revendication 1, **caractérisé en ce que** (IV) est mis en oeuvre en excès allant jusqu'à 10 % ou en défaut allant jusqu'à 10 % et (VI) en excès de 5 à 30 %.

10. Procédé selon la revendication 1, **caractérisé en ce que** la solution éventuellement aqueuse de (IV) contient la quantité de (VI) nécessaire pour neutraliser le chlorure d'hydrogène formé dans la réaction et **en ce que** l'on titre avec cette combinaison jusqu'à ce que, en 10 min, on consomme moins de 0,5 % de la quantité totale théoriquement nécessaire.
